(19) Europäisches Patentamt
European Patent Office
Office européen des brevets

(11) **EP 1 902 080 B1**

(12) **EUROPEAN PATENT SPECIFICATION**

(45) Date of publication and mention of the grant of the patent:
**24.02.2010 Bulletin 2010/08**

(21) Application number: **06754617.6**

(22) Date of filing: **29.06.2006**

(51) Int Cl.:
***C08F 257/02*** *(2006.01)* ***G01N 33/50*** *(2006.01)*
***G01N 31/16*** *(2006.01)*

(86) International application number:
**PCT/EP2006/006287**

(87) International publication number:
**WO 2007/003327 (11.01.2007 Gazette 2007/02)**

(54) **CARBOXYLATED LATEX PARTICLES**

CARBOXYLIERTE LATEXTEILCHEN

PARTICULES DE LATEX CARBOXYLÉ

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.07.2005 EP 05014340**

(43) Date of publication of application:
**26.03.2008 Bulletin 2008/13**

(73) Proprietors:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AT BE BG CH CY CZ DK EE ES FI FR GB GR HU IE IS IT LI LT LU LV MC NL PL PT RO SE SI SK TR**

(72) Inventors:
- **GEIGER, Albert**
  **82377 Penzberg (DE)**
- **FRACKMANN, Gernot**
  **68689 Grasellenbach/Hammelbach (DE)**
- **MORITZ, Hans-Ulrich**
  **21227 Bendestorf (DE)**
- **SLUKA, Peter**
  **82362 Weilheim (DE)**

(56) References cited:
**EP-A- 0 476 528 WO-A-95/14700**

EP 1 902 080 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The present invention relates to processes for preparing carboxylated latex particles based on the copolymerization of a monoalkenylaromatic monomer (A) and an aliphatic unsaturated carboxylic acid (B), where the copolymerization takes place at a pH in the range from pH > 1.5 to pH < 4.5. The pH is monitored during the copolymerization process and regulated by suitable compensatory titration. The invention likewise relates to the carboxylated latex particles obtainable by one of the processes according to the invention, and to the use of these particles in an immunological test method.

Surface-modified latices have been employed for more than 40 years in many biomedical areas of application, such as, for example, in diagnostic tests as support material, inter alia for enzyme immobilization. Singer, J.M. and Plotz, C.M. (American Journal of Medicine 21 (1956) 888-896) used latex particles as long ago as 1956 in the development of homogeneous immunoassays.

Besides aminated latex particles, the latices mainly employed as starting material in biomedical applications have been functionalized on the surface for example by carboxyl, epoxide or hydroxyl groups. Biopolymers such as, for example, specific antibodies are linked to these latex particles either by passive coating (adsorption) or by covalent bonding directly or with the aid of activating reagents such as, for example, N-hydroxybenzotriazole.

Many processes for preparing latex particles are known in the art.

For example, Roncari, G., (US 4,226,747) describes a process for preparing latices which consist predominantly of styrene and butadiene and which are distinguished by anilylsulpho groups on the latex surface. The co-monomers employed are sodium methallylsulphonate, acrylic acid and itaconic acid. The resulting latex is reacted with sodium dihexyl sulphosuccinate in water, $\alpha,\alpha'$-azobisisobutyramidinium chloride in water and p,p'-dithiobisaniline in styrene.

The application of Yaacoub, E.J., (DE 102 04 234) relates to a polymer latex prepared using at least one carbohydrate or carbohydrate derivative which is substituted by a group capable of free-radical polymerization and has surfactant properties. The polymer latex prepared by this process preferably comprises styrene and methyl methacrylate as comonomers. These latex particles can be employed in cosmetic, clinical or diagnostic applications.

Bowell, S.T. et al., (US 2,734,883) prevented the decrease in pH during the emulsion copolymerization of styrene and butadiene, despite the acid produced by decomposition of the peroxo initiator, by continuous or intermittent addition of alkali so that the pH is kept at or above 9.7.

In Gasche, H.E. et al., (US 2003/0153678), the pH is adjusted to a pH of 9-11 before addition of the initiator in the batch polymerization of a latex, and is monitored during the reaction so that it does not deviate by more than $\pm$ 0.5.

In the polymerization process for a polybutadiene latex filed by Sturt, A.G. and Feast, A.A.J. (DE 2 103 610), the pH is monitored up to a conversion of 15% in order to ensure emulsion-stable emulsion conditions.

To prepare an impact-resistant nitrile polymer in a multistage process of Takahashi, A., et al., (JP 61166813), the pH is adjusted to below 5 in the first copolymerization which is carried out batchwise, and the pH is reduced to 3 before the second, semi-continuous, stage.

A further process for preparing a stable latex was described by Sun-Lin, C. et al. (EP 0 476 528). Polymerization of the latex is initially carried out in the presence of a surface-active phosphate ester at a pH of below 3.5. The latex particles are then adjusted to a pH of between 7 and 10.5 by adding ammonia solution.

In the patent application of Meiners, C. et al., (DE 102 36 395), an aqueous polymer dispersion is obtained in a batch emulsion polymerization and is preferably used as rust-preventing paint. The emulsion is in this case adjusted to a pH of greater than or equal to 4.5, preferably 5 - 7, with aqueous ammonium hydroxide solution.

Brewer, J.F., (US 3,032,524) developed a process in order to adjust the pH of a latex accurately. In this case, the pH is adjusted exactly to the desired pH with a tolerance of $\pm$ 0.1 pH units by means of carbon dioxide during the preparation process. For this purpose, carbon dioxide is fed into the reactor and finely distributed by means of an impeller agitator.

The use of latex particles, including the use of carboxylated latex particles, for diagnostic purposes is known in principle. Thus, Fisk, R.T. (US 3,088,875) in 1959 described an immunological test intended to diagnose antigens or antibodies in body fluids. Pure polystyrene latices were employed as support material. These were known latices manufactured by Monsanto Chemical Company or Koppers Company Inc. with particle sizes of 150 - 200 nm. The latex particles were coated with 7-S $\gamma$-G globulin (now called class G immunoglobulin) in order to detect the so-called rheumatoid factor (RF), a 19-S $\gamma$-M protein (now called class M immunoglobulin) in human serum by aggregation of the latex particles. The RF binds to the coated immunoglobulin G. This results in aggregation of the latex particles.

A latex functionalized on the surface by epoxy groups was prepared by Batz, H.G. et al. (EP 0 054 685). This latex is employed as support material to which biological and/or immunologically active substances are covalently bonded directly or via a coupling agent. One or more of the monomers glycidyl methacrylate, glycidyl acrylate, glycidyl vinyl ether, glycidyl vinyl phthalate and 3,4-epoxybut(1)ene is/are used as monomer containing epoxide groups. This/these is/are copolymerized for example with styrene, dienes, acrylamide, methacrylamide, alkyl, hydroxyalkyl and aminoalkyl acrylate or methacrylate, vinyl ether, vinyl ester or N-vinylpyrrolidone.

A patent application by Seksui Chemical Co. Ltd. (JP 59179609) is concerned with the preparation of a latex for the diagnosis of antigens and antibodies, with the pH being varied during the polymerization. In this case, styrene and styrene sulphonate in water is mixed with an emulsifier and a persulphate as initiator and polymerized initially under alkaline and subsequently under acidic or neutral conditions.

The publication by Miraballes-Martinez, I. et al. (J. Biomater. Sci. Polymer Edn. 8 (1997) 765-777) describes the preparation of a latex which is functionalized by chloromethyl groups on the surface and is suitable for covalent coupling of immunoglobulin G (IgG). The chloromethyl-styrene latex was in this case prepared in a two-stage process in a batch reactor. The experiments carried out in this publication showed that the IgG-latex obtained in this way can be employed for applications in immunodiagnostic tests.

Functionalized latices, predominantly carboxylated latices, are employed in the process patented by Hager, H. (US 3,857,931).

In DE 27 12 044, Beskid, G. and Savard, E.V. describe the coupling of antibodies, antigenic substances or other biological materials to latex particles for serological tests. The Streptococcus group A antigen is conjugated, using a water-soluble carbodiimides, to the purified latex particles from this process.

Focella, A., et al. (DE 27 23 449) describe an agglutination test based on carboxylated latices and able to detect barbiturates.

Fischer, E.A. (DE 28 40 767) deals with the types of carboxylated latex support particles to which immunologically active materials such as, for example, primary amines, amino acids, peptides, proteins, lipo- and glycoproteins, sterols, steroids, lipoids, nucleic acids, enzymes, hormones, vitamins, polysaccharides and alkaloids can be coupled. Typical suitable latices are according to this carboxylated styrene-butadienes, carboxylated polystyrenes, acrylic acid polymers, methacrylic acid polymers, acrylonitrile polymers, acrylonitrile-butadiene-styrenes, polyvinyl acetate-acrylates, polyvinylpyridines, vinyl chloride-acrylates and the like.

A further patent by Fischer, E.A. (US 4,264,766) describes the development of an immunological test in which a carboxylated styrene-butadiene latex (Dow CL 241) is activated with 1-amino-2-hydroxypropyl-dextran and further processed for the test.

Gallati, H. (DE 27 49 956) used a carboxylated styrene-butadiene latex to which an immunological reagent, e.g. HCG (human chorionic gonadotropin), is bound.

As explained above, latex particles can in principle be employed in diagnostic test methods. However, very great demands are made on the characteristics of the latices for use in diagnosis. In particular, it is very important for sensitive immunodiagnostic methods that the latex particles employed can be well characterized in terms of their size and/or in terms of their surface properties, in particular in terms of the charge distribution and charge density, and can be prepared reproducibly.

The defined and reproducible preparation of carboxylated latex particles and thus also the availability of carboxylated latex particles with defined charge distribution on the surface is difficult with prior art processes.

It was therefore an object of the present invention to establish processes which help to eliminate the disadvantages known from the prior art and provide carboxylated latex particles which can be employed for example advantageously in immunological test methods.

It has surprisingly been found a process for preparing carboxylated latex particles based on the copolymerization of a monoalkenylaromatic monomer (A) and an aliphatic unsaturated carboxylic acid (B), where the copolymerization takes place at a constant target pH in the range from pH > 1.5 to pH < 4.5, where the pH is monitored during the copolymerization process and regulated by suitable compensatory titration. Carboxylated latex particles with the desired size and charge density can be prepared very reproducibly in this process and the latex particles obtained by this process show particular advantages and properties.

## Detailed description of the invention

The present application relates to a process for preparing carboxylated latex particles based on the copolymerization of a monoalkenylaromatic monomer (A) and an aliphatic unsaturated carboxylic acid (B), where the copolymerization takes place at a constant target pH in the range from pH > 1.5 to pH < 4.5. The pH is monitored during the copolymerization process and regulated by suitable compensatory titration. It is preferred for the monitoring of the pH in the copolymerization mixture and the compensatory titration to take place continuously. It is further preferred for the pH regulation to take place continuously and automatically. It is possible under the strongly acidic pH conditions of the process according to the invention for the charge density of the carboxyl functions on the latex particles to be controlled reproducibly and in a targeted manner.

In a preferred embodiment, the copolymerization takes place at a pH of greater than or equal to pH 2.0 and less than pH 4.0.

In a "copolymerization", two or more monomers are simultaneously polymerized together, the resulting copolymer being composed of the constituent monomers. The properties of the copolymers depend on the combination and

reactivity of the monomers, and the proportions of monomers and the management of the reaction. Copolymerizations can in principle be carried out in heterogeneous and homogeneous systems in reactors operated continuously, semicontinuously and batchwise. Copolymers with a wide variety of properties can be produced thereby. Although copolymers composed of three or more different monomers (ternary or quaternary copolymers etc.) exist, preferably not more than three different polymerizable monomers are employed.

Seed polymerization is a special emulsion polymerization process used in particular for preparing latices with a very narrow particle size distribution, and latices composed of more than one type of monomer and of defined microstructure within the individual latex particles. It is carried out by adding the monomer to be polymerized to a dispersion of latex particles of a uniform particle size distribution, called the seed latex. The monomer diffuses uniformly into the seed latex particles and is polymerized therein. In the specific case of seed polymerization, the concentration of the emulsifier present in the emulsion is kept within the critical micelle concentration (CMC). Otherwise, newly produced, much smaller latex particles would lead to a great broadening of the size distribution. Seed polymerization corresponds according to the model of Smith, W.V. and Ewart, R.H. (Journal of Chemical Physics 16 (1948) 592 - 599) to phases II and III of an emulsion polymerization. The particle formation phase (I) of an emulsion polymerization is redundant due to the presence of the seed latex particles.

Numerous publications have appeared since 1950 on the theory of particle growth in seed polymerization (inter alia Poehlein, G.W. and Vanderhoff, J.W., J. Polymer Sci. 11 (1973) 447 - 452; Feeney, P.J., Napper, D.H., Gilbert, R.G., J. Colloid Interface Sci. 118 (1987) 493 - 505). The theoretical descriptions of latex preparation, based on the Smith-Ewart theory, deal with the influence of various factors on the particle size distribution. Poehlein and Vanderhoff describe therein for example "competitive growth" leading to a narrower particle size distribution. In this connection, they assume that, owing to differences in the specific surface area, smaller particles of the seed latex grow faster than larger ones, because there is a greater probability of monomers diffusing into the smaller particles.

Monodisperse latices with larger particle diameters are usually prepared by carrying out stepwise polymerizations. This entails initially a first seed latex being prepared by suitable polymerization. These seed latex particles then serve as starting material in the subsequent (seed) polymerization. It is possible in this way to prepare latices with a very narrow particle size distribution. Such polymerizations are frequently carried out as batch polymerizations, with the rate at which the monomers enter the seed particles, the average number of free radicals in the latex particle and the volume growth rate being decisive for the influence on the particle size distribution. In the industrial preparation of latices with particular size distributions, such as, for example, in the styrene-butadiene rubber, seed polymerizations are frequently employed with the reaction being managed semicontinuously or semibatchwise.

Semibatch emulsion polymerization is preferably employed industrially when the aim is to obtain aqueous dispersions. For this purpose, two different so-called feed methods are used, the monomer feed and the emulsion feed.

In the monomer feed, the initial charge comprises water, emulsifier and initiator, and usually a small proportion of the monomer, and the remaining monomer and possibly also initiator are added during the polymerization.

In the emulsion feed, for example the initial charge comprises a portion of the emulsion and, after the reaction has started, the remaining monomer emulsion, which may differ in composition from the initially charged emulsion, is metered into the reactor.

The advantage of semibatchwise management of the reaction is that the polymerization rate and the heat of evolution can be controlled via the feed rate, the concentration of unreacted monomer can be kept low, and the reaction mixture can be cooled with cold feed. In order to produce a uniform polymer in the copolymerization of two monomers differing in reactivity, it is possible either for the initial charge to comprise an excess of the monomer which polymerizes more slowly, and to feed in the more reactive monomer, or for the monomer composition to be adjusted during the feeding (Chujo, K., et al., J. Polymer Sci. 27 (1969) 321-332; Snuparek, J. and Krška, F., J. of Applied Polymer Science 20 (1976)1753-1764). To control the copolymer composition, various monomer dispensing strategies have been developed and quantified in order to set the necessary, time-dependent metering rates (Hamielec, A.E., et al., Compr. Polym. Sci 3 (1989) 17-31). It is thus possible by suitable feed conditions to vary the degree of polymerization, the particle size and the particle size distribution without altering the overall composition of the latex. If the feed rate is not too high, according to Krackeler, J.J. and Naidus, H., J. Polymer Sci. 27 (1969) 207-235, a quasi steady-state is set up with monomers which comply which the Smith-Ewart mechanism, i.e. no formation of new particles takes place during the monomer metering, with the net reaction rate R and the feed rate F being approximately identical, and R depending only on the feed rate (Wessling, R.A., J. of Applied Polymer Science 12 (1968) 309 - 319). The monomer concentration [M] in this case assumes a value below the saturation concentration and changes only slightly during the feed period.

In this case, the reaction rate R [mol/s] and the monomer metering rate F [mol/s] can be described by the following equation of the formula I.

Formula I:

$$\frac{1}{R} = G + \frac{1}{F}$$

In this, the constant G depends on the average number of free radicals per particle, the number of particles, the growth rate constants, the reactivity coefficients and the partition coefficients of the monomers between the latex and the aqueous phase.

The time-conversion plot for semibatchwise polymerizations is linear over a wide range, and the molar mass distribution or the composition of the polymer becomes homogeneous because a substantially constant polymerization rate is maintained over the reaction time (Snuparek, J. and Krška, F., J. of Applied Polymer Science 20 (1976) 1753-1764).

It is important and preferred for the copolymerization to proceed in a controlled manner under so-called "starved conditions".

It has proved to be particularly advantageous to construct the carboxylated latex particles according to the invention according to the core-shell principle. In this process, the initial charge comprises a core or seed latex, and the shell with the desired properties is prepared by copolymerization on this seed latex. Suitable in principle as seed latex are all latex particles. It is preferred for the seed latex to be able to enter into covalent connections with the reagents employed to construct the carboxylated shell. The seed latex preferably employed is a polystyrene mixed latex or a pure polystyrene latex. The skilled person is able to select suitable seed latices without an inventive step. The skilled person is able to calculate from the size and the concentration of seed latex particles the amounts of monoalkenylaromatic monomer which are required to form the desired shell. The amount of aliphatically unsaturated carboxylic acid can be ignored in the calculation and adjustment of the particle size, because the proportionate amount thereof is negligible in this context.

The final size or target size of the particles depends substantially on the number and size of the seed latex particles and on the amount of monoalkenylaromatic monomer present. The relation between particle size, target molecule, weight of target latex and weight of seed latex is represented by formula II:

Formula II:

$$DT = DS \times (wtot/wseed)^{1/3}$$

where:

- *DT = average diameter of the target latex
- *DS = average diameter of the seed latex (average)
- *wtot = wseed + wmonomer in g
- *wseed = weight of seed latex employed in g
- *monomer = weight of monomer employed in g

The number (amount) and size of the seed particles thus specifies the amount of monomers necessary to achieve a particular target particle size of the final product.

The mixture for a stepwise polymerization is normally chosen so that the target latex results as an approximately 3 to 30% strength suspension. The conditions are preferably chosen so that the target latex is in the form of a 5 to 20% strength suspension.

The monoalkenylaromatic monomer (A) will typically be a styrene, an alpha-methylstyrene or a vinyltoluene. Preferred monoalkenylaromatic monomers are styrene and alpha-methylstyrene. If necessary, it is also possible to employ a mixture of two or more monoalkenylaromatic monomers in the process according to the invention. However, preferably not more than two of these monomers are mixed. Styrene is preferably used as monoalkenylaromatic monomer.

The aliphatically unsaturated carboxylic acid (B) will preferably be acrylic acid, methacrylic acid, fumaric acid, itaconic acid, maleic acid or maleic anhydride. If necessary, it is also possible to employ a mixture of two or more aliphatically unsaturated carboxylic acids in the process according to the invention. However, preferably not more than two of these aliphatically unsaturated carboxylic acids will be employed. It is further preferred for the aliphatically unsaturated carboxylic acid to be selected from the group consisting of acrylic acid, methacrylic acid, fumaric acid and itaconic acid. Acrylic acid is the preferred aliphatic.unsaturated carboxylic acid.

It is necessary in many processes for preparing latex particles to add the various monomers employed for the copolymerization separately to the reaction mixture. A further advantage of the process according to the invention is that a monoalkenylaromatic monomer (A) and the aliphatic unsaturated carboxylic acid (B) can be matched with one another in such a way that (B) dissolves in (A). The aliphatic unsaturated carboxylic acid (B) is therefore preferably dissolved in the monoalkenylaromatic monomer (A) and, in the process according to the invention, the mixture of (A) and (B) is fed into the reaction mixture.

As mentioned, it is particularly advantageous that the process according to the invention for preparing carboxylated latex particles proceeds at a strongly acidic pH and that this pH is continuously measured and kept constant during the reaction. The desired pH for the reaction conditions is also referred to as target pH. A constant pH for the chosen reaction conditions in the context of the invention described herein is present when the deviation of the pH in the copolymerization mixture from the desired target pH amounts to a pH of 0.3 or less. In the process according to the invention the pH in the copolymerization mixture does not deviate by more than pH 0.3 from the target pH. The process according to the invention is accordingly **characterized in that** the pH in the copolymerization mixture deviates by a maximum of ± pH 0.3 from the target pH. The process according to the invention is preferably **characterized in that** the pH in the copolymerization mixture deviates by a maximum of ± pH 0.2 or likewise preferably by a maximum of ± pH 0.1 from the target pH.

It is possible in principle to use any suitable alkaline solution to adjust the pH. The pH-regulating reagent preferably employed is an aqueous solution of an alkali metal carbonate, bicarbonate or hydroxide.

As appreciated in the prior art, there are various possibilities for dealing with the problem of the poor solubility of (forming) latex particles. It has proved appropriate in the process according to the present invention for the copolymerization to be carried out in aqueous emulsion. In a further preferred embodiment, the process according to the invention is thus **characterized in that** the copolymerization takes place in aqueous emulsion. This copolymerization can moreover preferably take place in the absence of emulsifiers.

The (co)polymerization reaction as is characteristic for the production of latex particles can be initiated by various agents. Known in the art are in particular gamma rays and reagents which release free radicals. The use of reagents which form free radicals under suitable conditions is preferred according to the invention. Peroxide-based free-radical formers are typically employed. Preference is given to water-soluble peroxides, in particular persulphates and peroxides, such as, for example, sodium or ammonium persulphate and hydrogen peroxide.

The carboxylated latex particles which can be prepared by the process according to the invention are particularly distinguished also by the fact that the charge density on the surface of these particles can be predetermined and reproduced in a targeted manner. The charge density is ascertained by means of atomic force microscopy (AFM) (Tan, S., et al., Langmuir 21 (2005) 43-49). The so-called parking area is stated as a measure of the charge density. The parking area indicates, for example for the carboxylated latex particles according to the invention, the average area occupied by a carboxyl group on the latex surface.

It is possible in the process according to the invention, as shown in Example 3, to adjust a particular, desired charge density by suitable choice of the reaction parameters. The carboxylated latex particles preferably prepared by the process according to the invention have an average parking area of from 10 to 160 $(10^{-10}\ m)^2$, likewise preferably from 20 to 130 $(10^{-10}\ m)^2$, or likewise preferably from 30 to 120 $(10^{-10}\ m)^2$.

It is possible for the parking area not only to be adjusted empirically but also to be calculated in advance by formula III.

Formula III:

$$y = ax^b$$

The y axis of the function describes the parking area in $(10^{-10}\ m)^2$, and the x axis represents the acrylic acid concentration set in the formulation in $10^{-1}$ mol/l. This value corresponds to the total concentration of acrylic acid in the emulsion achieved during the metering.
When the pH = 2, then: $y = 15.99x^{-0.9805}$
When the pH = 3, then: $y = 41.065x^{-0.4706}$
When the pH = 4, then: $y = 51.061x^{-0.4722}$

The values for a and b in formula III can be ascertained empirically for each polymerization mixture and at each pH. The charge density is preferably set in accordance with formula III in the process according to the invention.

Since the carboxylated latex particles according to the invention have an exactly defined charge density, they are very particularly suitable for use in diagnostic methods. The defined charge density has the effect that the carboxylated

latex particles show a uniform and reproducible behaviour.

The present invention thus includes in a preferred embodiment also the carboxylated latex particles prepared according to the invention.

The carboxylated latex particles prepared by the process according to the invention are outstandingly suitable for covalent attachment of biochemical molecules.

Carboxylated latices are activated for example by using carbodiimides as coupling reagents. Biomolecules can be linked to the latex via amide groups directly or via an active ester. The coupling reagents used in this connection are preferably water-soluble carbodiimides (WSC). The following commercially available, water-soluble carbodiimides are particularly suitable for activation and coupling of biomolecules to carboxylated latex particles: 1-cyclohexyl-3-(2-morpholinoethyl)carbodiimides metho-p-toluenesulphonate and 1-ethyl-3-(3-dimethylaminopropyl)carbodiimide hydrochloride. These coupling agents can be employed for direct linkage of biomolecules via amide groups. However, it is likewise possible for free carboxyl functions initially to be converted into an active ester in order then to undertake the coupling via this active ester (e.g. an O-acylurea intermediate). Both procedures are depicted diagrammatically below.

a) Reaction for direct coupling to carboxylated latex:

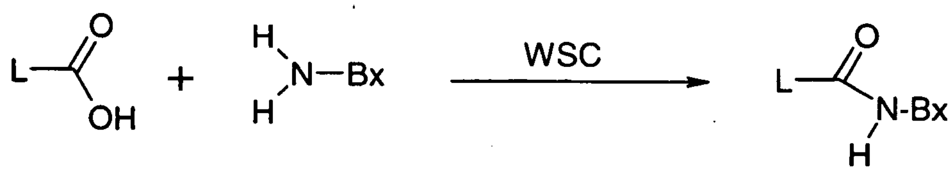


carboxylated latex biomolecule activated latex

b) Activation of carboxylated latices via an active ester:

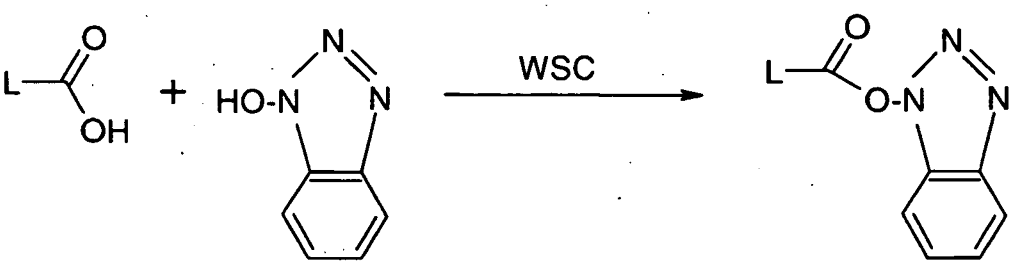


carboxylated latex 1-benzo-triazolol active ester

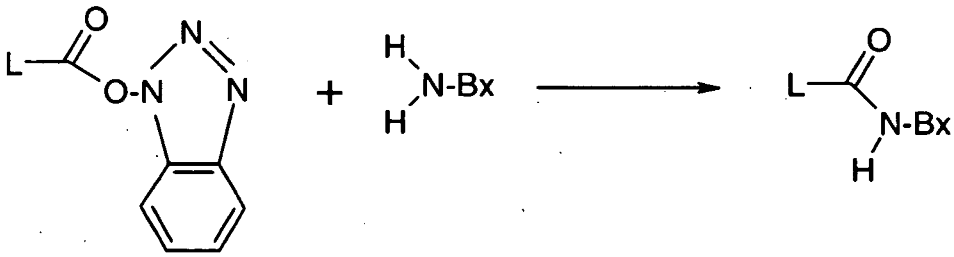

active ester biomolecule activated latex

One advantage of the coupling via an active ester is that the carbodiimides can be removed before the antigen or antibody is coupled to the latex, and thus has no influence on the immunological test.

Suitable biomolecules are all molecules employed in conventional diagnostic test methods. They particularly preferably include nucleic acids, peptides and proteins. However, it is also possible advantageously to employ other biomolecules such as, for example, haptens, lipids or polysaccharides.

Preferred biomolecules are from the group of proteins, especially those proteins which represent good binding partners for further biomolecules, with very particular preference in this connection for lectins, avidin, streptavidin and antibodies.

The term "antibody" means, besides the intact immunoglobulins, also all antibody fragments. These include for example Fab, Fab' or $F(ab')_2$ fragments. The term antibody without the addition of "monoclonal" or "polyclonal" always includes both types of antibodies, plus chimeric constructs and all fragments listed above.

After attachment of the biomolecules to the carboxylated latex particles according to the invention, the skilled person refers to biomolecule-latex conjugates or latex conjugates for short. The latex conjugates based on carboxylated latex particles according to this invention likewise represent a preferred embodiment of the invention.

The latex conjugates based on carboxylated latex particles according to this invention are particularly advantageously employed in immunodiagnostic test methods. The design and procedure for such test methods are familiar to the skilled person.

The following examples further illustrate the invention characterized in the appended claims.

## Example 1

### Preparation of seed latex particles (seed latex)

A seed latex consisting of completely polymerized and emulsifier-stabilized latex particles is prepared by an emulsion polymerization. The procedure for this follows for example the experimental method of van den Brink (M. v.d. Brink: "On-line monitoring of polymerization reactions by Raman spectroscopy, application to control of emulsion copolymerizations and copolymerization kinetics", Technische Universiteit Eindhoven, 2000).

The stated amount of sodium dodecyl sulphate (SDS) and $NaHCO_3$ is weighed into a 1000 ml glass beaker and dissolved in the stated amount of water by stirring. The solution is cautiously flushed with nitrogen. The solution is transferred together with the styrene into a 1150 ml laboratory reactor with jacket and anchor stirrer. After the reaction mixture has reached a constant temperature, the prepared initiator solution is added.

The seed is prepared at a reaction temperature of 80°C with a stirring speed of 300 rpm.

**Table 1:**

| Exemplary formulation for a seed preparation according to v.d. Brink in the emulsion polymerization of styrene | |
|---|---|
| **Seed preparation according to v.d. Brink** | |
| Component | Content in % by mass |
| Styrene | 27.06 |
| Emulsifier (sodium dodecyl sulphate) | 2.88 |
| Buffer (sodium bicarbonate) | 0.09 |
| Initiator (sodium peroxodisulphate) | 0.19 |
| Continuous phase (deionized water) | 69.78 |

In the emulsion copolymerization, in each case one part of the monoalkenylaromatic monomer (e.g. styrene) is replaced by the seed polymer latex of Example 1. The amount of monomer necessary to achieve a particular target particle size can be determined according to formula I with the aid of the particle concentration and size present in the seed.

## Example 2

### pH-Controlled copolymerization under strongly acidic conditions

The advantage of this method is that the surface charge can be adjusted to the particular requirements by targeted setting of the pH and appropriate choice of the acrylic acid concentration.

### General procedure for carrying out the pH-controlled copolymerization:

The styrene employed is destabilized by distillation before use, and the demineralized water is degassed with a stream of nitrogen. In the examples specifically detailed hereinafter, the styrene was divided into 2 portions, and the amounts listed in the table were (a) added to the seed latex (monomer feed), or (b) metered continuously into the reaction mixture (emulsion feed). The further components were employed directly without any processing.

The stated amount of $NaHCO_3$ is weighed into a 1000 ml glass beaker and dissolved in the stated amount of

water by stirring. The solution is cautiously flushed with nitrogen. The solution is transferred with the seed emulsion and with the weighed amount of styrene (a) into a 1150 ml laboratory reactor. After the reaction mixture has reached a constant temperature of 72°C, the prepared initiator solution is added. The styrene (b) and the acrylic acid are added by means of metering pumps (piston diaphragm pump, Prominent Gamma 4 type). The sodium bicarbonate solution used to adjust the pH is metered in with a perfusor or likewise with a metering pump.

The stated amount of styrene (b) is metered in together with the acrylic acid dissolved therein over a period of 240 min. A latex is produced with a core of polystyrene and a shell of styrene-acrylic acid copolymer with an average particle size of about 125 - 135 nm diameter.

The latex was prepared at a reaction temperature of 72°C with a stirring speed of 180 rpm.

In the copolymerizations carried out, a sodium bicarbonate solution was added through a second metering line to set and regulate the stated pH values. Table 2 shows the initial weights in the exemplary formulations for pH-controlled copolymerization of the carboxylated latices:

**Table 2:**

| Exemplary formulations for pH-controlled preparation of carboxylated latices | | |
|---|---|---|
| **Component** | **Initial weight [g]** | **Initial weight [wt%]** |
| Polystyrene seed 26.9% (39 nm) | 9.57 | 1.15-1.165 |
| Initial amount of styrene (a) | 2.57 | 0.31-0.313 |
| Sodium bicarbonate (initial amount) | 0.01 - 0.05 | $1.2 - 1.6 \cdot 10^{-3}$ |
| Initiator (sodium peroxodisulphate | 1 | 0.12 |
| Continuous phase (water) | 740.0 | 89.39-90.082 |
| Styrene (b) (metered over 4 h) | 68.03 | 8.218-8.281 |
| Acrylic acid (metered over 4 h) | 0.706-7.06 | 0.853-0.086 |
| Sodium peroxodisulphate (metered over 4 h) | 1 | 0.12 |
| Sodium bicarbonate to adjust the pH during the reaction | 0.24-1.14 | 0.029-0.14 |

**Example 3**

**Preparation of various carboxylated latices**

In the semibatchwise reactions in Table 3, the pH was kept constant during the metering time at the stated pH values of respectively pH = 2, pH = 3 and pH = 4, and the influence of the pH during the reaction on the parking area was investigated.

The pH was adjusted with a sodium bicarbonate solution which was continuously metered in by means of a perfusor during the monomer metering. The concentration of the $NaHCO_3$ solution depends on the amount of acrylic acid to be metered and is between 0.25 and 1.15 mol/l, and the total amount of metered sodium bicarbonate under the described experimental conditions is 2.9-13.5 mmol, depending on the amount of acrylic acid and pH.

At pH 2, sodium peroxodisulphate was additionally metered over a period of 240 min, because the acid-catalyzed hydrolysis of the initiator is no longer negligible at this pH.

The initial weights of the polystyrene seed and of the initial charge of styrene and metered styrene, of the continuous phase (demineralized water) and of the initiator employed are described in Table 2. Table 3 below shows besides the exact amount of acrylic acid employed, broken down according to the particular pH set, also the resulting parking area of the latex particles. The particle size of the target latex particles was found to be 125 - 135 nm.

**Table 3:**

| Resulting parking area in the pH-controlled preparation of carboxylated latices | | | |
|---|---|---|---|
| **Batch** | **Mass of acrylic acid [g]** | **Acrylic acid concentration [$10^{-1}$ mol/l]** | **parking area [$10^{-10}$m]$^2$** |
| pH=2 | | | |
| 1 | 0.706 | 0.132 | 107.6 |
| 2 | 0.884 | 0.166 | 100.8 |

(continued)

| Resulting parking area in the pH-controlled preparation of carboxylated latices | | | |
|---|---|---|---|
| Batch | Mass of acrylic acid [g] | Acrylic acid concentration [$10^{-1}$ mol/l] | parking area [$10^{-10}$m]$^2$ |
| pH=2 | | | |
| 3 | 1.009 | 0.189 | 78.1 |
| 4 | 1.765 | 0.331 | 51.1 |
| 5 | 7.06 | 1.32 | 11.8 |
| pH=3 | | | |
| 6 | 0.706 | 0.132 | 130.5 |
| 7 | 1.009 | 0.189 | 83.5 |
| 8 | 1.07 | 0.201 | 76.8 |
| 9 | 1.765 | 0.331 | 65.2 |
| 10 | 7.06 | 1.32 | 38.2 |
| pH=4 | | | |
| 11 | 0.706 | 0.132 | 151.5 |
| 12 | 1.009 | 0.189 | 116.5 |
| 13 | 1.479 | 0.277 | 99.1 |
| 14 | 1.765 | 0.331 | 72.5 |
| 15 | 7.06 | 1.32 | 47.6 |

As the amount of acrylic acid falls there is an exponential increase in the parking area, while there is a linear decrease in the mass of metered acrylic acid relative to styrene. In the experiments in which the pH was kept constant at pH = 4, less acrylic acid is incorporated on the latex surface by contrast with a constant pH of pH = 3. More acrylic acid is present in protonated form in the acidic range, and is thus more hydrophobic and copolymerizes more readily with the styrene. In this case, more acrylic acid is incorporated in the copolymerization than dissolves in the styrene.

As is evident from Table 3, carboxylated latex particles can be produced with a desired, predetermined charge density in a very targeted manner with the aid of the present process.

**Example 4**

**Reproduction batch**

It was attempted in this experiment to reproduce the particles from batch 2 in Example 3.

**Table 4:**

| Parking area on repetition of batch 2 (at pH 2) | | | |
|---|---|---|---|
| Batch | Mass of acrylic acid [g] | Acrylic acid concentration [$10^{-1}$ mol/l] | Parking area ($10^{-10}$m)$^2$ |
| 16 | 0.884 | 0.166 | 98.5 |

As is evident from Table 4, virtually identical reproduction of batch 2 was possible in new batch 16. The described method of pH-controlled emulsion copolymerization to prepare carboxylated latex particles is thus also distinguished by optimal reproducibility.

## Example 5

### Preparation of an anti-CRP-latex conjugate

15 mg of latex were activated in 0.75 ml of 20 mM MES (2-(N-morpholino)ethanesulphonic acid) buffer of pH 6.1 in the presence of 4 mM sulpho-NHS (N-hydroxysuccinimide) and 4 mM EDC (1-ethyl-3-(3-dimethylaminopropyl) carbodiimides hydrochloride) and incubated in a roller incubator at room temperature for 1 h. Then, 705 µl of a MAb<CRP> solution (C=0.96 mg of MAb/ ml) (Mab<CRP> = monoclonal antibody against C-reactive protein (CRP)) in 20 mM MES buffer of pH 6.1 were added and incubation was continued for 20 min. Subsequently, 45 µl of a 2% strength Synperonic solution in MES of pH 6.1 were added. The reaction mixture was incubated for a further 100 min and the reaction was stopped by adding 30 µl of a 2M glycine HCl solution (pH 11). The conjugate mixture was centrifuged, and the supernatant was removed and redispersed in buffer (50 mM glycine HCl, pH 8.0 with 0.03% Synperonic and 0.05% sodium azide).

The prepared conjugates were assessed using the Roche Diagnostics Cobas Mira system. The performance of the conjugates based on the latices prepared according to the invention complied with the requirements for use in a homogeneous immunoassay for detecting CRP.

### Claims

1. Process for preparing carboxylated latex particles based on the copolymerization of a monoalkenylaromatic monomer (A) and an aliphatic unsaturated carboxylic acid (B), where the copolymerization takes place at a constant target pH in the range from pH > 1.5 to pH < 4.5, where this target pH is monitored during the reaction and kept constant by compensatory titration and wherein the pH in the copolymerization mixture deviates by a maximum of ± pH 0.3 from the target pH.

2. Process according to Claim 1, **characterized in that** the monoalkenylaromatic monomer (A) is styrene or alpha-methylstyrene.

3. Process according to Claim 1 or 2, **characterized in that** the aliphatically unsaturated carboxylic acid (B) is acrylic acid, methacrylic acid, fumaric acid or itaconic acid.

4. Process according to any of Claims 1 to 3, **characterized in that** the copolymerization takes place in aqueous emulsion.

5. Process according to any of Claims 1 to 4, **characterized in that** the copolymerization is triggered by free radicals.

6. Process according to any of Claims 1 to 5, **characterized in that** a suitable seed latex is added before the copolymerization.

7. Process according to any of Claims 1 to 6, **characterized in that** monomer (B) is dissolved in monomer (A).

8. Process according to Claim 7, **characterized in that** an aqueous solution of an alkali metal carbonate, bicarbonate or hydroxide is employed as pH-regulating reagent.

9. Use of carboxylated latex particles obtainable by a process according to any of claims 1 to 8 in an immunological test method.

### Patentansprüche

1. Verfahren zur Herstellung carboxylierter Latex-Partikel basierend auf der Co-Polymerisation eines monoalkenylaromatischen Monomers (A) und einer aliphatischen ungesättigten Carbonsäure (B),
wobei die Co-Polymerisation bei einem konstanten Ziel-pH-Wert im Bereich von pH > 1,5 und pH < 4,5 erfolgt und wobei dieser Ziel-pH-Wert während der Reaktion gemessen und durch Gegentitration konstant gehalten wird, und wobei der pH-Wert im Co-Polymerisationsansatz maximal um ± pH 0,3 vom Ziel-pH-Wert abweicht.

2. Verfahren nach Anspruch 1, **dadurch gekennzeichnet, dass** das monoalkenylaromatische Monomer (A) Styrol oder alpha-Methyl-Styrol ist.

3. Verfahren nach Anspruch 1 oder 2, **dadurch gekennzeichnet, das** die aliphatisch ungesättigte Carbonsäure (B) Acrylsäure, Methacrylsäure, Fumarsäure oder Itaconsäure ist.

4. Verfahren nach einem der Ansprüche 1 bis 3, **dadurch gekennzeichnet, dass** die Co-Polymerisation in wässriger Emulsion erfolgt.

5. Verfahren nach einem der Ansprüche 1 bis 4, **dadurch gekennzeichnet, dass** die Co-Polymerisation durch Radikale getriggert wird.

6. Verfahren nach einem der Ansprüche 1 bis 5, **dadurch gekennzeichnet, dass** vor der Co-Polymerisation ein geeigneter Saat-Latex zugegeben wird.

7. Verfahren nach einem der Ansprüche 1 bis 6, **dadurch gekennzeichnet, dass** das Monomer (B) in Monomer (A) gelöst wird.

8. Verfahren nach einem der Ansprüche 7, **dadurch gekennzeichnet, dass** als pH-regulierendes Reagenz eine wässrige Lösung eines Alkalicarbonats, -hydrogencarbonats oder -hydroxids eingesetzt wird.

9. Verwendung carboxylierter Latex-Partikel erhältlich nach einem Verfahren nach einem der Ansprüche 1 bis 8 in einem immunologischen Testverfahren.

**Revendications**

1. Procédé pour préparer des particules de latex carboxylé, qui se base sur la copolymérisation d'un monomère monoalcénylaromatique (A) et d'un acide carboxylique aliphatique insaturé (B), dans lequel la copolymérisation a lieu à un pH cible constant dans la plage d'un pH > 1,5 à un pH < 4,5, ce pH cible étant surveillé au cours de la réaction et étant maintenu constant par titrage compensatoire, et dans lequel le pH, dans le mélange de copolymérisation, dévie d'une valeur maximale de ± pH 0,3 par rapport au pH cible.

2. Procédé selon la revendication 1, **caractérisé en ce que** le monomère monoalcénylaromatique (A) est le styrène ou l'alpha-méthylstyrène.

3. Procédé selon la revendication 1 ou 2, **caractérisé en ce que** l'acide carboxylique aliphatique insaturé (B) est l'acide acrylique, l'acide méthacrylique, l'acide fumarique ou l'acide itaconique.

4. Procédé selon l'une quelconque des revendications 1 à 3, **caractérisé en ce que** la copolymérisation a lieu en émulsion aqueuse.

5. Procédé selon l'une quelconque des revendications 1 à 4, **caractérisé en ce que** la copolymérisation est déclenchée par des radicaux libres.

6. Procédé selon l'une quelconque des revendications 1 à 5, **caractérisé en ce qu'**on ajoute un latex d'ensemencement approprié avant la copolymérisation.

7. Procédé selon l'une quelconque des revendications 1 à 6, **caractérisé en ce que** le monomère (B) est dissout dans le monomère (A).

8. Procédé selon la revendication 7, **caractérisé en ce qu'**on utilise une solution aqueuse d'un carbonate, d'un bicarbonate ou d'un hydroxyde de métal alcalin, à titre d'agent de régulation du pH.

9. Utilisation de particules de latex carboxylé, que l'on peut obtenir via un procédé selon l'une quelconque des revendications 1 à 8, dans un procédé de test immunologique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4226747 A, Roncari, G.
- DE 10204234, Yaacoub, E.J.
- US 2734883 A, Bowell, S.T.
- US 20030153678 A, Gasche, H.E.
- DE 2103610, Sturt, A.G. and Feast, A.A.J.
- JP 61166813 B, Takahashi, A.
- EP 0476528 A, Sun-Lin, C.
- DE 10236395, Meiners, C.
- US 3032524 A, Brewer, J.F.
- US 3088875 A, Fisk, R.T.
- EP 0054685 A, Batz, H.G.
- JP 59179609 B
- US 3857931 A, Hager, H.
- DE 2712044, Beskid, G. and Savard, E.V.
- DE 2723449, Focella, A.
- DE 2840767, Fischer, E.A.
- US 4264766 A, Fischer, E.A.
- DE 2749956, Gallati, H.


### Non-patent literature cited in the description


- **Singer, J.M. ; Plotz, C.M.** *American Journal of Medicine,* 1956, vol. 21, 888-896
- **Miraballes-Martinez, I. et al.** *J. Biomater. Sci. Polymer Edn.,* 1997, vol. 8, 765-777
- **Smith, W.V. ; Ewart, R.H.** *Journal of Chemical Physics,* 1948, vol. 16, 592-599
- **Poehlein, G.W. ; Vanderhoff, J.W.** *J. Polymer Sci.,* 1973, vol. 11, 447-452
- **Feeney, P.J. ; Napper, D.H. ; Gilbert, R.G.** *J. Colloid Interface Sci.,* 1987, vol. 118, 493-505
- **Chujo, K. et al.** *J. Polymer Sci.,* 1969, vol. 27, 321-332
- **Hamielec, A.E. et al.** *Compr. Polym. Sci,* 1989, vol. 3, 17-31
- **Krackeler, J.J. ; Naidus, H.** *J. Polymer Sci.,* 1969, vol. 27, 207-235
- **Wessling, R.A.** *J. of Applied Polymer Science,* 1968, vol. 12, 309-319
- **Tan, S. et al.** *Langmuir,* 2005, vol. 21, 43-49
- **M. v.d. Brink.** On-line monitoring of polymerization reactions by Raman spectroscopy, application to control of emulsion copolymerizations and copolymerization kinetics. Technische Universiteit Eindhoven, 2000